# EUROPEAN PATENT APPLICATION

(11) **EP 4 194 431 A1**
(43) Date of publication of application: **14.06.2023**
(21) Application number: 21852894.1
(22) Date of filing: 30.07.2021
(51) Int. Cl.: C07C 29/74, C07C 29/76, C07C 31/20

(54) **METHOD FOR REFINING BIO-BASED CRUDE ETHYLENE GLYCOL**

(30) Priority: 03.08.2020 CN 202010766596
(71) Applicant: Changchun Meihe Science and Technology Development Co., Ltd., Changchun, Jilin 130102 (CN)
(72) Inventor: YUAN, Yi, Changchun, Jilin 130102 (CN)
(74) Representative: Abthorpe, Mark
(86) International application number: PCT/CN2021/109536
(87) International publication number: WO 2022/028319

(57) **Abstract**

A method for refining a bio-based crude ethylene glycol, the method comprising: using a bio-based crude ethylene glycol as a raw material, diluting same with water to an ethylene glycol concentration of 1-95 weight%; under the conditions of the temperature being 0-100°C and the volume space velocity being 0.01-20 BV/hr, continuously passing the diluted ethylene glycol aqueous solution through an adsorption bed, which is filled with one or more macroporous adsorption resins and an optional ion exchange resin, for an adsorption treatment so as to obtain a purified ethylene glycol aqueous solution; and then dehydrating same to obtain ethylene glycol.

## Description

### Technical Field

The present invention relates to a bio-based crude ethylene glycol, and particularly to a method for refining a bio-based crude ethylene glycol that contains hydroxyl impurities having boiling points close to that of ethylene glycol, such as butanediol, pentanediol, hexanediol and optional 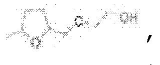 and trace amounts of impurities affecting the ultraviolet transmittance of ethylene glycol, such as acids, ethers, aldehydes, ketones, compounds containing double bonds, and/or alcohols.

### Background Art

Due to the oil price uncertainty and increasing awareness of sustainability, rapid development has taken place in recent years in the technology for producing ethylene glycol using biomass as raw material. However, due to different synthesis routes, the ethylene glycol production process based on the biomass route results in the production of hydroxyl-containing byproducts that are different from those produced from the petroleum route or coal route, and impurities that affect the ultraviolet transmittance of ethylene glycol and are present in trace amounts or even at levels below the detection limit of GC, such as acids, ethers, aldehydes, ketones and/or alcohols. The traditional purification method for liquid compounds is the rectification process that separates substances based on their different boiling points. However, the boiling points of these impurities are close to that of ethylene glycol, and impurities that affect the ultraviolet transmittance of ethylene glycol and are present in trace amounts or even at levels below the detection limit of GC, such as acids, ethers, aldehydes, ketones and/or alcohols exhibit physical properties similar to those of ethylene glycol and boiling points close to that of ethylene glycol. Therefore, separating ethylene glycol from these alcohol impurities by direct rectification would lead to a low yield of ethylene glycol, and high energy consumption. In addition, ethylene glycol obtained by rectification still contains trace amounts of impurities, so the ultraviolet transmittance of ethylene glycol cannot meet the requirements for the fiber grade and bottle grade polyester.

US4935102, US4966658, US5423955, and US8906205 describe processes for separating ethylene glycol from butanediol by using different azeotropic agents. The azeotropic agent and ethylene glycol have an azeotropic point. In general, the temperature of the azeotropic point is obviously lower than the boiling point of ethylene glycol. The boiling point of the azeotrope containing ethylene glycol and the azeotropic agent is thus significantly different from the boiling points of impurities, such as butanediol, so ethylene glycol and butanediol can be economically separated through rectification.

The ethylene glycol production process based on the biomass route results in the production of alcohol impurities other than butanediol that have boiling points very close to that of ethylene glycol, such as pentanediol, hexanediol and optionally 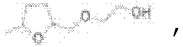 and impurities that affect the ultraviolet transmittance of ethylene glycol and are present in trace amounts or even at levels below the detection limit of GC, such as acids, ethers, aldehydes, ketones and/or alcohols. The aforesaid patent applications do not indicate that the processes therein can effectively separate these impurities.

CN106946654A describes the use of an adsorption bed containing a porous carbon adsorbent for adsorbing impurities in biomass ethylene glycol to refine ethylene glycol. This technique only describes the increase in the ultraviolet transmittance of ethylene glycol but does not indicate its use for separating alcohol impurities, such as butanediol, the compound of the following molecular formula: 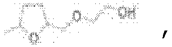 pentanediol or hexanediol.

CN201010200038.5 describes a method for refining ethylene glycol by using zeolite and aluminum silicate as the adsorbents. However, this method only describes that 1,2-butanediol can be effectively removed by the adsorbent but does not indicate that the adsorbent can improve the ultraviolet transmittance or other hydroxyl impurities.

CN201110047173.5 describes a method for increasing the ultraviolet transmittance of ethylene glycol by having it contact an adsorbent contained in a fixed bed to retain the substances affecting the ultraviolet transmittance of ethylene glycol in the fixed bed. However, this method only describes that the adsorbent bed increases the ultraviolet transmittance but does not indicate its effect in terms of adsorbing hydroxyl-containing alcohol impurities, such as butanediol, pentanediol, and hexanediol.

The traditional direct adsorption process that uses adsorbents for producing petroleum-based ethylene glycol or coal-based ethylene glycol cannot effectively separate special impurities in bio-based ethylene glycol.

### Summary of the Invention

The present invention provides a method for refining a bio-based crude ethylene glycol to cost-efficiently separate, with a high yield, the bio-based crude ethylene glycol from hydroxyl-containing impurities having boiling points close to that of ethylene glycol and trace amounts of impurities affecting the ultraviolet transmittance of ethylene glycol, such as acids, ethers, aldehydes, ketones, compounds containing double bonds, and/or alcohols, thereby increasing the purity and ultraviolet transmittance of ethylene glycol.

The method for refining bio-based crude ethylene glycol of the present invention comprises: using a bio-based crude ethylene glycol as a raw material, diluting it with water to an ethylene glycol concentration of 1-95 wt%, preferably 20-90 wt%, more preferably 40-85 wt%, and under the conditions of a temperature of 0-100°C, preferably 10-80°C, particularly preferably 10-50°C, and a volume space velocity of 0.01-20 BV/hr, preferably 0.01-10 BV/hr, continuously passing the diluted ethylene glycol aqueous solution through an adsorption bed, which is filled with one or more, preferably one or two macroporous adsorption resins and an optional ion exchange resin, for an adsorption treatment to obtain a purified ethylene glycol aqueous solution, and then dehydrating it to obtain ethylene glycol. The purity and ultraviolet transmittance of the dehydrated ethylene glycol meet the industrial standard for the premium grade product of polyester grade ethylene glycol. The standard for the premium grade product of polyester grade ethylene glycol herein refers to a purity of 99.9% or higher, and an ultraviolet transmittance of 75%, 95% and 99% or higher at a wavelength of 220 nm, 275 nm and 350 nm, respectively.

The resin is able to separate ethylene glycol from trace amounts of impurities affecting the ultraviolet transmittance, such as acids, ethers, aldehydes, ketones, compounds containing double bonds, and/or alcohols, and hydroxyl-containing impurities that affect the purity of ethylene glycol and have boiling points close to that of ethylene glycol, such as alcohol impurities of butanediol, pentanediol, hexanediol and optional 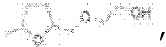 to increase the ultraviolet transmittance and purity of ethylene glycol cost-efficiently with a high yield.

Optionally, the bio-based crude ethylene glycol is subjected to an ultraviolet lamp pretreatment process, and for example, the bio-based crude ethylene glycol is irradiated under ultraviolet light at a wavelength of not less than 100 nm, preferably not less than 180 nm, more preferably 180-350 nm. In a partially bio-based crude ethylene glycol raw material, the presence of larger amounts of impurities affecting ultraviolet transmittance leads to a low ultraviolet transmittance, which in turn results in a shortened resin regeneration cycle as well as increased regeneration cost and resin cost. Therefore, to extend the resin regeneration cycle and reduce the resin operation cost, it is desirable to add a controllable ultraviolet lamp process before the resin adsorption process to increase the ultraviolet transmittance of ethylene glycol. Without degrading ethylene glycol molecules, the controllable ultraviolet light can convert impurities of compounds that contain double bonds and absorb suitable ultraviolet light (such as 180-350 nm ultraviolet light) into compounds that contain no double bonds, thereby preliminarily increasing the ultraviolet transmittance of ethylene glycol.

The ultraviolet lamp pretreatment process may be performed in the following manner: the bio-based crude ethylene glycol raw material is introduced into a container containing an ultraviolet lamp with a wavelength of not less than 100 nm, preferably not less than 180 nm, more preferably 180-350 nm, at a temperature of 0-170°C, preferably 10-120°C, more preferably 10-50°C, and retained therein for more than 0-2 h, preferably 0.1-1 h. The ultraviolet lamp is preferably a low pressure mercury lamp, a medium pressure mercury lamp, a high pressure mercury lamp, a LED lamp, a high intensity discharge lamp, or a metal halide lamp. After its ultraviolet transmittance is preliminarily increased, the discharged material can be further treated in the resin adsorption process.

The macroporous adsorption resin is, for example, preferably a macroporous adsorption resin containing styrene and/or divinylbenzene as the backbone, more preferably a macroporous adsorption resin containing styrene and/or divinylbenzene as the backbone with a specific surface area greater than 800 m²/g.

The ion exchange resin may be a weakly basic anion exchange resin, preferably a weakly basic anion exchange resin containing primary amine, secondary amine, and/or tertiary amine groups on its surface.

The bio-based crude ethylene glycol refers to ethylene glycol prepared from biomass (herein biomass preferably refers to a first-generation edible biomass including corn and sugar cane, and a second-generation non-grain biomass obtained from agricultural and forestry wastes including crop stalk, wood, and bagasse). In addition to ethylene glycol, the bio-based crude ethylene glycol contains but is not limited to butanediol, pentanediol, and hexanediol. Optionally, the bio-based crude ethylene glycol also contains the compound of the following molecular formula: 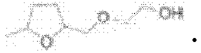 The butanediol is preferably 1,2-butanediol, 2,3-butanediol, 1,4-butanediol. The pentanediol is preferably 1,2-pentanediol. The hexanediol is preferably 1,2-hexanediol. More preferably, the bio-based crude ethylene glycol contains but is not limited to:
88-100 wt% ethylene glycol, preferably 95-100 wt% ethylene glycol, more preferably 98-100 wt% ethylene glycol (exclusive of the endpoint 100 wt%),
0-5 wt%, preferably 0-1 wt%, more preferably 0-0.5 wt%, particularly preferably 0-0.1 wt% of butanediol (preferably 1,2-butanediol, 2,3-butanediol, and/or 1,4-butanediol, exclusive of the endpoint 0),
0-5 wt%, preferably 0-1 wt%, more preferably 0-0.5 wt%, particularly preferably 0-0.1 wt% of pentanediol (preferably 1,2-pentanediol, exclusive of the endpoint 0),
0-5 wt%, preferably 0-2 wt%, more preferably 0-1.5 wt% of hexanediol (preferably 1,2-hexanediol, exclusive of the endpoint 0), and
optionally 0-5 wt%, preferably 0-1 wt%, more preferably 0-0.5 wt%, particularly preferably 0-0.1 wt% of

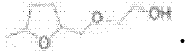

The bio-based crude ethylene glycol also optionally comprises:
0-5 wt%, preferably 0-1 wt%, more preferably 0-0.1 wt% of 1,2-propylene glycol, and
0-5 wt%, preferably 0-1 wt%, more preferably 0-0.1 wt% of diethylene glycol.

Since the bio-based crude ethylene glycol contains specific hydrophilic hydroxyl-containing impurities, the dilution with water facilitates the adsorption of the aforesaid impurities.

The water may be, for example, desalted water.

The dehydration may be achieved by, for example, rectification.

Since trace amounts of impurities affecting the ultraviolet transmittance of ethylene glycol, such as acids, ethers, aldehydes, ketones, compounds containing double bonds, and/or alcohols can absorb ultraviolet light even at trace levels, these impurities cannot be detected by an instrument or a chemical method, such as gas chromatography, or liquid chromatography. Therefore, their contents in the raw material can be represented only by ultraviolet transmittance. In the present application, due to the presence of the aforesaid impurities, the ultraviolet transmittance of the bio-based crude ethylene glycol raw material at 220 nm, 275 nm, and 350 nm fails to meet at least one requirement specified for the premium grade product of polyester grade ethylene glycol, and such a transmittance may be expressed as follows for example: ultraviolet transmittance at 220 nm of less than 75%, preferably less than 40%, more preferably less than 10%; ultraviolet transmittance at 275 nm of less than 95%, preferably less than 70%, more preferably less than 30%; and/or ultraviolet transmittance at 350 nm of less than 99%, preferably less than 97%, more preferably less than 96%.

By using the method of the present invention, the purity of ethylene glycol can be increased to 99.9% or higher at a high yield with an ethylene glycol recovery rate of 99.9% or higher, and the ultraviolet transmission at 220 nm, 275 nm, and 350 nm can be increased to 75%, 95%, and 99% or higher, respectively. In addition, the cost of the present invention is significantly lower than that of the traditional rectification method.

### Description of drawings

Fig. 1 is the process flow chart of Example 1.
Fig. 2 is the change curve of ultraviolet transmittance of the ethylene glycol product according to Example 1 (Fig. 2a) and change curve of ethylene glycol content (Fig. 2b).
Fig. 3 is the process flow chart of Example 2.
Fig. 4 is the change curve of ultraviolet transmittance of the ethylene glycol product according to Example 2 (Fig. 4a) and change curve of ethylene glycol content (Fig. 4b).
Fig. 5 is the process flow chart of Example 3.
Fig. 6 is the change curve of ultraviolet transmittance of the ethylene glycol product according to Example 3 (Fig. 6a) and change curve of ethylene glycol content (Fig. 6b).
Fig. 7 is the process flow chart of Comparative Example 1.
Fig. 8 is the change curve of ultraviolet transmittance of the ethylene glycol product according to Comparative Example 2 (Fig. 8a) and change curve of ethylene glycol content (Fig. 8b).

### Specific Embodiments

### Examples

The present invention is further described by referring to the examples below, but the present invention is not limited to the following examples.

### Example 1

The resin column was filled with 200 ml of XA-1G macroporous adsorption resin, purchased from Xi'an Sunresin New Materials Co., Ltd., as an adsorbent, and the backbone of the resin was styrene-divinylbenzene with a specific surface area of 1200 m²/g.

The crude ethylene glycol product, which was obtained by biomass hydrogenation and rectification for preliminary removal of light and heavy fractions, was used as the raw material. The raw material was analyzed by using the analytic methods specified in the national standard GB/T4649-2008, and the contents of various components were as follows: 99.700 wt% of ethylene glycol, 0.020 wt% of 1,2-pentanediol, 0.250 wt% of 1,2-hexanediol, 0.010 wt% of 1,2-butanediol, 0.010 wt% of 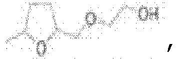 and 0.010 wt% of other components. The ultraviolet transmittance of the raw material was 13.5% at 220 nm, 59.0% at 275 nm, and 96.8% at 350 nm.

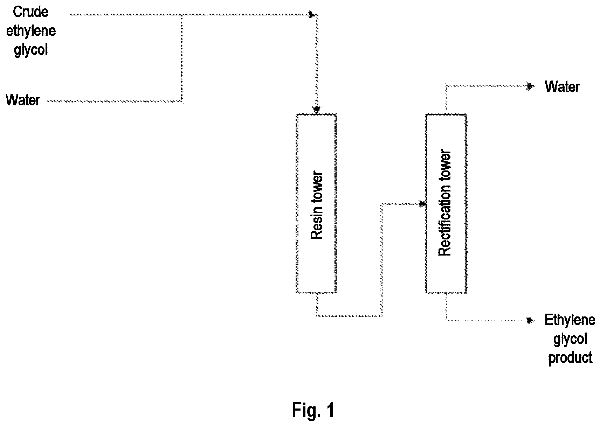

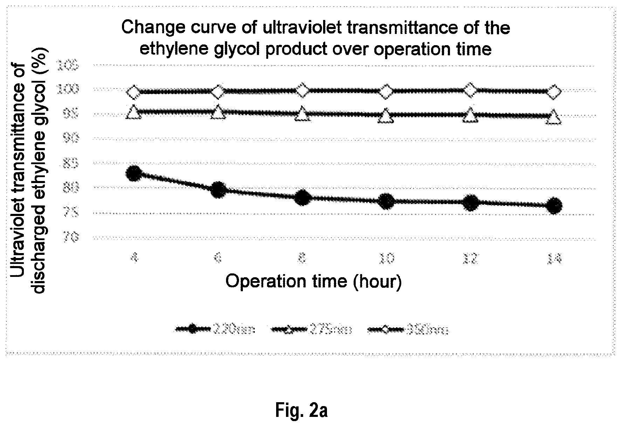

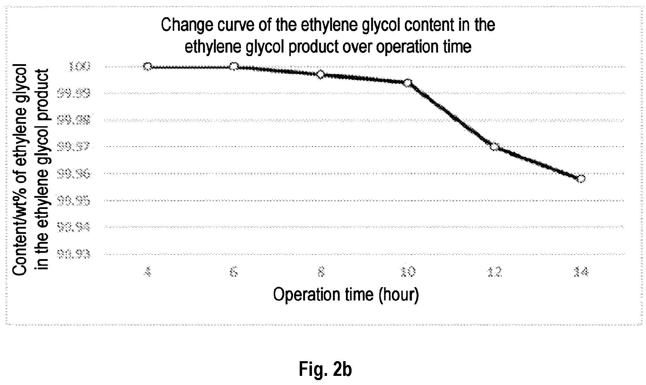

According to the process flow as shown in Fig. 1, the crude ethylene glycol raw material and desalted water were mixed at a mass ratio of 3:1 to obtain a crude ethylene glycol aqueous solution, which was then continuously introduced into the resin bed at 30°C and a volume space velocity of 0.5 BV/h, and the material discharged from the resin bed was dehydrated by using a rectification tower. Fig. 2a and Fig. 2b show the ultraviolet transmittance and purity results of the ethylene glycol product obtained after the adsorption and dehydration treatments.

After 14 hours, the ultraviolet transmittance at 275 nm was reduced to a level below the requirement specified for the premium grade product, indicating that the adsorbent was ineffective. A total of 1524.6 g of crude ethylene glycol aqueous solution containing 1140.0 g of ethylene glycol was treated; 1139.7 g of an acceptable ethylene glycol product was finally obtained. Therefore, the yield of the refined acceptable ethylene glycol was 99.97%.

### Comparative Example 1

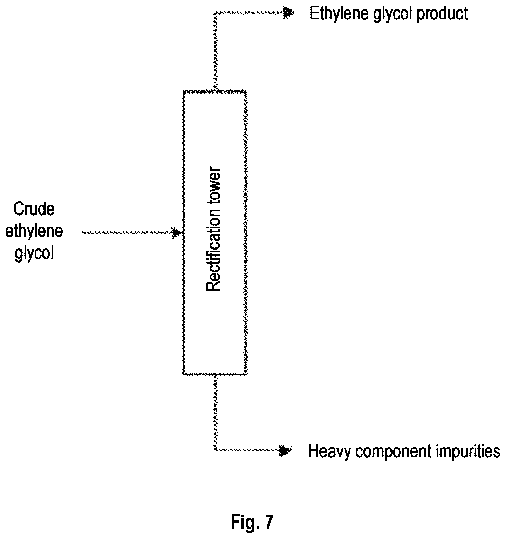

The crude ethylene glycol product described in Example 1, which was obtained by biomass hydrogenation and rectification for preliminary removal of light and heavy fractions, was used as the raw material and was separated by using the traditional rectification method shown in Fig. 7. The total number of theoretical plates of the rectification tower was 90, the reflux ratio was 15:1, and the operating pressure was 10 kPa (absolute) . The raw material was introduced into the rectification tower from the 40th theoretical plate at a flow rate of 200 g/h. The ethylene glycol product was withdrawn from the top of the rectification tower at a flow rate of 195 g/h. The contents of ethylene glycol, 1,2-butanediol, 1,2-pentanediol, 1,2-hexanediol and 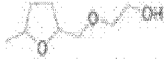 in the ethylene glycol product, as weight percentages, were: 99.920%, 0.010%, 0.020%, 0.040%, and 0.010%, respectively. The ultraviolet transmittance was 20.8% at 220 nm, 63.2% at 275 nm, and 98.5% at 350 nm. The total rectification yield of ethylene glycol was 97.7%.

Test results show that the traditional rectification process could effectively separate 1,2-hexanediol from ethylene glycol to reach an ethylene glycol purity of 99.90% or higher, but the yield of ethylene glycol was only 97.7%. In addition, the high reflux ratio led to high energy consumption of steam and failed to effectively increase the ultraviolet transmittance; in contrast, the method of the present invention could increase the purity of ethylene glycol to 99.90% or higher in a high-yield and low-cost manner, and the ethylene glycol ultraviolet transmittance at 220 nm, 275 nm, and 350 nm was increased to over 75%, 95% and 99%, respectively.

### Comparative Example 2

The crude ethylene glycol product described in Example 1, which was obtained by biomass hydrogenation and rectification for preliminary removal of light and heavy fractions, was used as the raw material, and the same type and volume of adsorbent as described in Example 1 was packed into the resin column.

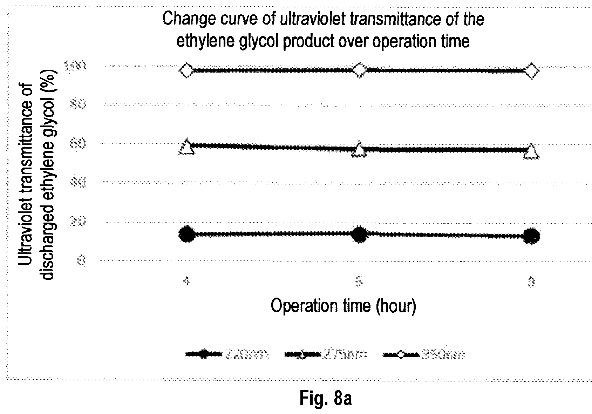

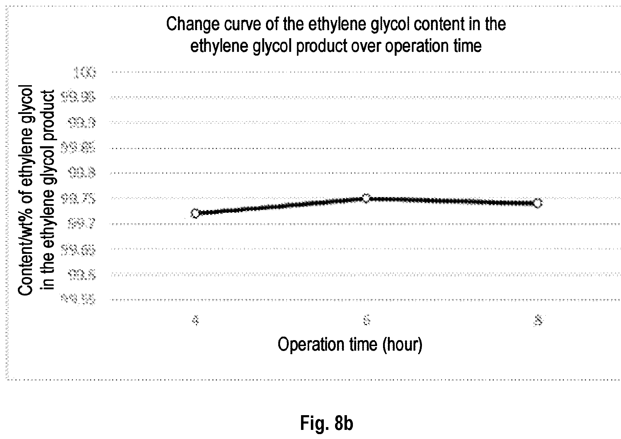

According to the process flow as shown in Fig. 1, the crude ethylene glycol raw material was not mixed with water but was continuously introduced into the resin bed at 30°C and a volume space velocity of 0.5 BV/h. Fig. 8a and Fig. 8b show the ultraviolet transmittance and purity results of the ethylene glycol product obtained after the adsorption treatment.

Test results show that in Comparative example 2, despite the use of the same raw material, adsorbent and operation conditions as those described in Example 1, due to the failure to mix the bio-based crude ethylene glycol with water, the special impurities in the bio-based crude ethylene glycol were not effectively adsorbed by the adsorbent, so the purity and ultraviolet transmittance of the discharged ethylene glycol were not increased to the levels as specified in the standard for the premium grade product.

### Example 2

A low pressure mercury ultraviolet lamp with a wavelength of 254 nm and a power of 23 W was placed into an ultraviolet lamp container with a capacity of 30 ml. The resin column A was filled with 750 ml of D303 weakly basic anion exchange resin containing primary amine groups with styrene-divinylbenzene as the backbone, purchased from Xi'an Sunresin New Materials Co., Ltd., as the adsorbent. The resin column B was filled with 200 ml of L493 macroporous adsorption resin purchased from Dow Chemical as the adsorbent. The backbone of the macroporous adsorption resin was macroporous styrene polymer and its specific surface area was 1100 m²/g.

The crude ethylene glycol product, which was obtained by biomass hydrogenation and rectification for preliminary removal of light and heavy fractions, was used as the raw material. The raw material was analyzed by using the analytic methods specified in the national standard GB/T4649-2008, and the contents of various components were as follows: 99.753 wt% of ethylene glycol, 0.036 wt% of 1,2-pentanediol, 0.146 wt% of 1,2-hexanediol, 0.033 wt% of 1,2-butanediol, 0.010 wt% of 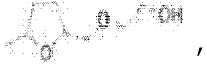 0.002 wt% of 1,4-butanediol, 0.010 wt% of diethylene glycol, and 0.010 wt% of other components; the ultraviolet transmittance of the raw material was 1.1% at 220 nm, 25.0% at 275 nm, 95.0% at 350 nm.

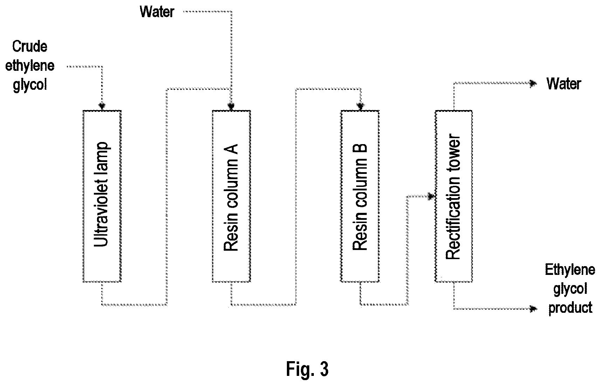

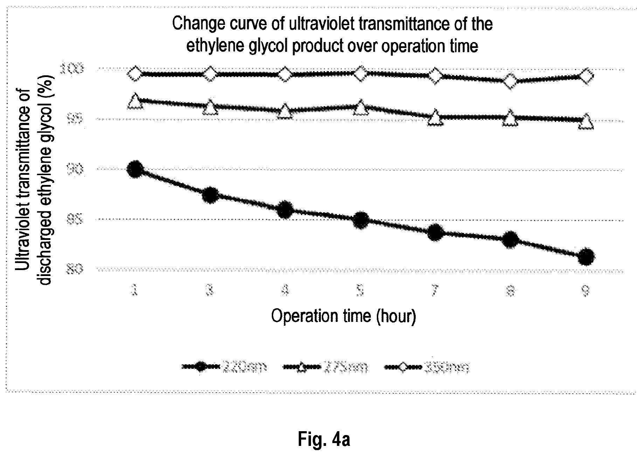

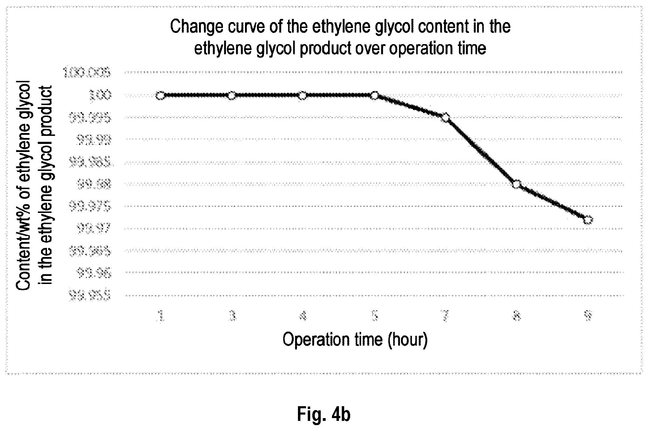

According to the process flow as shown in Fig. 3, the crude ethylene glycol raw material was continuously introduced into an ultraviolet lamp container at 20°C and a flow rate of 100 ml/h. The material discharged from the ultraviolet lamp container was mixed with desalted water at a mass ratio of 2:1 to obtain a crude ethylene glycol aqueous solution, which was then continuously introduced into the resin bed A at 20°C and a volume space velocity of 0.2 BV/h. The material discharged from the resin bed A was continuously introduced into the resin bed B at a volume space velocity of 0.75 BV/h with other conditions remaining unchanged; the material discharged from the resin bed B was dehydrated by using a rectification tower. Fig. 4a and Fig. 4b show the ultraviolet transmittance and purity results of the ethylene glycol product obtained after the ultraviolet lamp treatment and adsorption and dehydration treatments.

After 9.3 hours, the ultraviolet transmittance at 275 nm was reduced to a level below the requirement specified for the premium grade product, indicating that the adsorbent was ineffective. A total of 1038.8 g of crude ethylene glycol containing 1036.2 g of ethylene glycol was treated; 1036.0 g of acceptable ethylene glycol product was finally obtained. Therefore, the yield of the refined ethylene glycol was 99.98%.

### Example 3

The resin column A was filled with 40 ml of D303 weakly basic anion exchange resin containing primary amine groups with styrene-divinylbenzene as the backbone, purchased from Xi'an Sunresin New Materials Co., Ltd., as the adsorbent; the resin column B was filled with 200 ml of XA-1G macroporous adsorption resin purchased from Xi'an Sunresin New Materials Co., Ltd., as the adsorbent. The backbone of the macroporous adsorption resin was styrene-divinylbenzene and its specific surface area was 1200 m²/g.

The crude ethylene glycol product, which was obtained by biomass hydrogenation and rectification for preliminary removal of light and heavy fractions, was used as the raw material. The raw material was analyzed by using the analytic methods specified in the national standard GB/T4649-2008, and the contents of various components were as follows: 98.81 wt% of ethylene glycol, 0.10 wt% of 1,2-pentanediol, 1.05 wt% of 1,2-hexanediol, 0.02 wt% of 1,2-butanediol, 0.01 wt% of 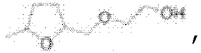 and 0.01 wt% of other components; the ultraviolet transmittance of the raw material was 6.6% at 220 nm, 63.0% at 275 nm, 95.0% at 350 nm.

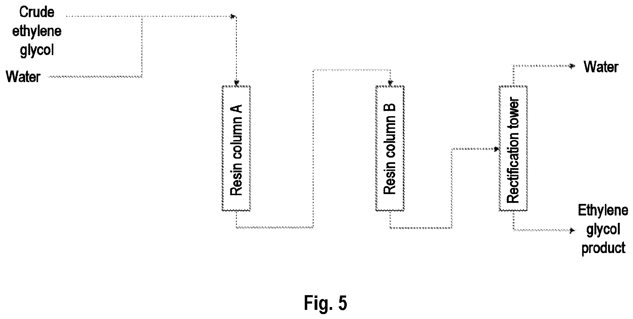

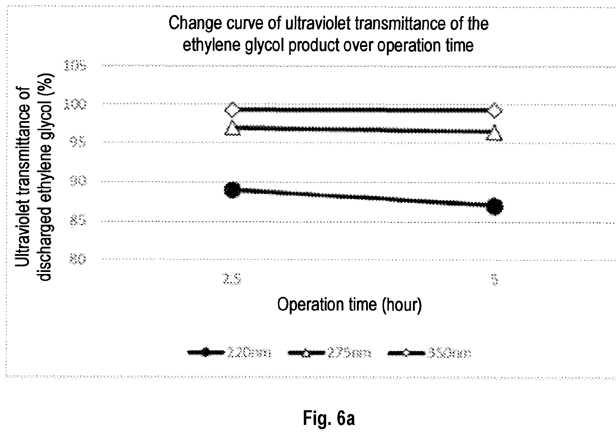

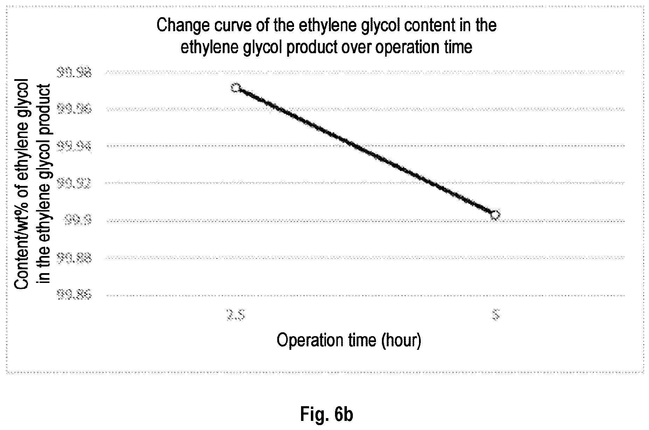

As shown in Fig. 5, the crude ethylene glycol raw material was mixed with desalted water at a mass ratio of 3:1 to obtain a crude ethylene glycol aqueous solution, which was then continuously introduced into the resin bed A at 30°C and a volume space velocity of 1.0 BV/h. The material discharged from the resin bed A was continuously introduced into the resin bed B at a volume space velocity of 0.2 BV/h with other conditions remaining unchanged; the material discharged from the resin bed B was dehydrated by using a rectification tower. Fig. 6a and Fig. 6b show the ultraviolet transmittance and purity results of the ethylene glycol product obtained after adsorption.

After 5 hours, the purity was reduced to a level below the requirement specified for the premium grade product, indicating that the adsorbent was ineffective. A total of 217.8 g of crude ethylene glycol aqueous solution containing 161.41 g of ethylene glycol was treated; 161.38 g of acceptable ethylene glycol product was finally obtained. Therefore, the yield of the refined ethylene glycol was 99.98%.

From the embodiments, it can be seen that the present invention can be used to effectively separate ethylene glycol from trace amounts of impurities affecting ultraviolet transmittance, such as acids, ethers, aldehydes, ketones, compounds containing double bonds, and/or alcohols, and hydroxyl-containing impurities that affect ethylene glycol purity and have boiling points close to that of ethylene glycol, so as to increase the ultraviolet transmittance and purity of ethylene glycol cost-efficiently with a high yield.

## Claims

1. A method for refining bio-based crude ethylene glycol, comprising: using a bio-based crude ethylene glycol as a raw material, diluting it with water to an ethylene glycol concentration of 1-95 wt%, preferably 20-90 wt%, more preferably 40-85 wt%, and under conditions of a temperature of 0-100°C, preferably 10-80°C, particularly preferably 10-50°C, and a volume space velocity of 0.1-20 BV/hr, preferably 0.01-10 BV/hr, continuously passing the diluted ethylene glycol aqueous solution through an adsorption bed, which is filled with one or more, preferably one or two macroporous adsorption resins and an optional ion exchange resin, for an adsorption treatment to obtain a purified ethylene glycol aqueous solution, and then dehydrating it to obtain ethylene glycol.

2. The method as claimed in claim 1, wherein the bio-based crude ethylene glycol is subjected to an ultraviolet lamp pretreatment process, and for example, the bio-based crude ethylene glycol is irradiated under ultraviolet light at a wavelength of not less than 100 nm, preferably not less than 180 nm, more preferably 180-350 nm.

3. The method as claimed in claim 2, wherein the ultraviolet lamp pretreatment process is performed in the following manner: the bio-based crude ethylene glycol as a raw material is introduced into a container containing an ultraviolet lamp with a wavelength of not less than 100 nm, preferably not less than 180 nm, more preferably 180-350 nm, at a temperature of 0-170°C, preferably 10-120°C, more preferably 10-50°C, and retained therein for more than 0-2 hours, preferably 0.1-1 hours.

4. The method as claimed in any one of claims 1 to 3, wherein the macroporous adsorption resin is a macroporous adsorption resin containing styrene and/or divinylbenzene as the backbone, more preferably a macroporous adsorption resin containing styrene and/or divinylbenzene as the backbone with a specific surface area greater than 800 m²/g.

5. The method as claimed in any one of claims 1 to 4, wherein the ion exchange resin is a weakly basic anion exchange resin, preferably a weakly basic anion exchange resin containing primary amine, secondary amine, and/or tertiary amine groups on its surface.

6. The method as claimed in any one of claims 1 to 5, wherein the bio-based crude ethylene glycol is ethylene glycol prepared from a biomass and contains butanediol, pentanediol and hexanediol in addition to ethylene glycol, and optionally, the bio-based crude ethylene glycol also contains a compound with a molecular formula of: 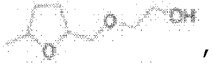 wherein the butanediol is preferably 1,2-butanediol, 2,3-butanediol, 1,4-butanediol, the pentanediol is preferably 1,2-pentanediol, and the hexanediol is preferably 1,2-hexanediol.

7. The method as claimed in any one of claims 1 to 6, wherein the bio-based crude ethylene glycol comprises:
88-100 wt% ethylene glycol, preferably 95-100 wt% ethylene glycol, more preferably 98-100 wt% ethylene glycol (exclusive of the endpoint 100 wt%),
0-5 wt%, preferably 0-1 wt%, more preferably 0-0.5 wt%, particularly preferably 0-0.1 wt% of butanediol (preferably 1,2-butanediol, 2,3-butanediol, and/or 1,4-butanediol, exclusive of the endpoint 0),
0-5 wt%, preferably 0-1 wt%, more preferably 0-0.5 wt%, particularly preferably 0-0.1 wt% of pentanediol (preferably 1,2-pentanediol; exclusive of the endpoint 0),
0-5 wt%, preferably 0-2 wt%, more preferably 0-1.5 wt% of hexanediol (preferably 1,2-hexanediol; exclusive of the endpoint 0), and
optionally 0-5 wt%, preferably 0-1 wt%, more preferably 0-0.5 wt%, particularly preferably 0-0.1 wt% of

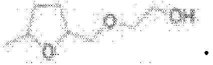

8. The method as claimed in any one of claims 1 to 7, wherein the bio-based crude ethylene glycol further optionally comprises:
0-5 wt%, preferably 0-1 wt%, more preferably 0-0.1 wt% of 1,2-propylene glycol, and
0-5 wt%, preferably 0-1 wt%, more preferably 0-0.1 wt% of diethylene glycol.

9. The method as claimed in any one of claims 1 to 8, wherein the dehydration is achieved by rectification.

10. The method as claimed in any one of claims 1 to 9, wherein the ultraviolet transmittance of the bio-based crude ethylene glycol raw material at 220 nm, 275 nm and 350 nm fails to meet at least one requirement specified for the premium grade product of polyester grade ethylene glycol.
